# EUROPEAN PATENT SPECIFICATION

(11) **EP 2 496 131 B1**
(45) Date of publication and mention of the grant of the patent: **27.05.2020**
(21) Application number: 10776833.5
(22) Date of filing: 21.10.2010
(51) Int. Cl.: A61B 5/00, A61B 8/13

(54) **BIOINFORMATION ACQUISITION APPARATUS**
VORRICHTUNG ZUR ERFASSUNG VON BIOINFORMATIONEN
APPAREIL D'ACQUISITION DE BIOINFORMATIONS

(30) Priority: 04.11.2009 JP 2009253068
(43) Date of publication of application: 12.09.2012
(73) Proprietor: Canon Kabushiki Kaisha, Tokyo 146-8501 (JP)
(72) Inventor: ICHIHARA, Shigeru, Tokyo 146-8501 (JP); KOBAYASHI, Shuichi, Tokyo 146-8501 (JP)
(74) Representative: TBK
(86) International application number: PCT/JP2010/006240
(87) International publication number: WO 2011/055501

(56) References cited:
- WO-A1-2009/050632
- JP-A- 2006 208 050
- US-A1- 2007 197 886
- US-A1- 2009 005 685
- US-A1- 2010 053 618

## Description

### Technical Field

The present invention relates to a bioinformation acquisition apparatus using light and in particular to a technology for enhancing optical energy use efficiency by re-irradiating a living body with a flux of light emitted outside from a living body through light diffusion inside the living body.

### Background Art

The research and development have been forwarded for visualizing information in a living body by using noninvasive near infrared rays having a high transmission factor for a living body, i.e., a test object. In vivo material such as water, fat and hemoglobin present in a blood vessel has a peculiar spectrum in a near infrared wavelength range. Consequently, attentions have been attracted for studies in which a spatial coefficient distribution of absorption originating from those components is visualized to acquire functional information in a living body.

Methods for detecting optical absorption in a living body includes, e.g. diffuse measurement (DOT: Diffuse Optical Tomography) in which a flux of light that has been transmitted through a living body is directly measured, and photoacoustic measurement (PAT: Photo Acoustic Tomography) using photoacoustic effect.

In PAT, pulsed light generated by a light source is radiated to a living body, and an in vivo tissue inside the living body absorbs optical energy of the pulsed light which propagates and is diffused therein, and the tissue produces an acoustical wave to be measured. That is, an elastic wave of a test site produced when a test site absorbs the radiated optical energy and expands instantaneously is received by a transducer and identified due to a difference in absorption coefficient of optical energy between the test site such as a tumor and tissue other than the test site. This detection signal can be analyzed and processed, providing an optical characteristics distribution in a living body, particularly an optical energy absorption density distribution. Since an ultrasonic wave has high straightness in a living body compared with light, a photoacoustic measurement apparatus for detecting an ultrasonic wave has a high spatial resolution.

US 2006/0184042 A proposes an apparatus as a bioinformation imaging apparatus using photoacoustic effect. In the apparatus disclosed in US 2006/0184042 A, light is collected to a desired area in a living body by a lens having a light-collecting function, and a photoacoustic signal is detected while efficiently using optical energy.

WO 2009/050632 A1 discloses an apparatus, systems and methods provided for production and integration of compact illumination schemes. More particularly, disclosed structures relate to apparatus/systems and methods for production of highly compact illumination schemes, whereby photoacoustic waves are induced in a target sample. Additionally, the disclosed apparatus/systems and methods are effective to produce compact and portable integrated transducer-illumination arrays. The apparatus disclosed generally includes at least one lighting source and a beam splitting assembly. The systems disclosed generally include one or more apparatus for the production of compact lighting schemes, an ultrasonic transducer assembly and means for coupling the one or more apparatus and US transducer assembly with a target sample.

US 2009/005685 A1 discloses an ultrasonic probe which includes a light irradiating portion configured to radiate light for generating ultrasonic waves from a light absorber, an ultrasonic transducing portion configured to transduce the ultrasonic waves to an electric signal, and a light guide member configured to introduce light from a light source to the light irradiating portion. A light irradiating region of the light irradiating portion is included within an ultrasonic receiving region of the ultrasonic transducing portion.

### Summary of Invention

The above exemplified conventional bioinformation imaging apparatus using photoacoustic effect can acquire a stronger acoustical wave signal by applying large optical energy to a living body.

However, the case using such photoacoustic effect has a problem that, since optical energy of a flux of light is attenuated before the light reaches a deep portion in a living body, a strength of an acoustical wave signal coming from the deep portion is lowered. And therefore, it is necessary to use a pulsed light source generator having high power as a light source to observe a deep portion. However, the pulsed light source generator is limited in output and furthermore expensive. Further, there also arises a problem that as the output is higher, it becomes unstable.

Therefore, to enhance observation capability in a deep portion, it is desirable to use optical energy more efficiently.

As an expected example of such efficient use of optical energy, backscattering light, which is a flux of light emitted out of the living body by diffusion effect in the living body, can be reused to improve the optical energy use efficiency.

That is, a flux of light radiated into a living body loses straightness due to a strong diffusion effect in the living body, and a part of the flux of light is radiated outside the living body from an irradiation area and adjacent areas due to backscattering. By reusing this emitted flux of light, it can be expected to enhance strength of a signal. On the one hand, in an approach disclosed in US 2006/0184042 A, a lens having a light-collecting function, as described above, collects light in a desired area of a living body.

According to such an approach, a flux of light can be directly radiated into a living body to enhance optical energy use efficiency, but this approach cannot efficiently function so as to reuse the backscattering light described above.

An objective of the present invention, in terms of the aforementioned problems, is to provide a bioinformation acquisition apparatus which can reuse a flux of light emitted outside from a test object due to diffusion effect acting in the test object when light is radiated to the test object, and can enhance use efficiency of the radiated light.

This object is achieved by a bioinformation acquisition apparatus according to claim 1. Advantageous developments are subject-matters of the dependent claims.

A bioinformation acquisition apparatus adapted in the following manner is provided.

The bioinformation acquisition apparatus is a bioinformation acquisition apparatus having a light source for radiating a flux of light to a test object and a detector for detecting a signal output based on radiation of the flux of light to the test object, including: a reflective member adapted to reflect a flux of light emitted out of the test object when the flux of light is radiated to the test object, wherein the reflective member is placed closer to an irradiation area of the flux of light on a surface of the test object than the light source so as to cover at least part of the irradiation area from above, and configured to be equal to or larger in size than the irradiation area.

A flux of light emitted outside from a test object when light is radiated to the test object is reflected by a reflective member and projected to the test object again, so that the flux of light emitted outside from the test object can be reused, improving use efficiency of the radiated light.

Further features will become apparent from the following description of examples with reference to the attached drawings.

### Brief Description of Drawings

[Fig. 1]Fig. 1 is a cross-sectional view of an example of a configuration of a bioinformation acquisition apparatus having a reflective member according to an example 1.
[Fig. 2]Fig. 2 is a top view illustrating the relation between the size of an irradiation area and the size of a reflective member in example 1.
[Fig. 3]Fig. 3 is a cross-sectional view of an example of a configuration of a bioinformation acquisition apparatus having a reflective member according to an example 2.
[Fig. 4]Fig. 4 is a cross-sectional view of an example of a configuration of a bioinformation acquisition apparatus having a reflective member according to an example 3.
[Fig. 5]Fig. 5 is a cross-sectional view of an example of a configuration of a bioinformation acquisition apparatus having a reflective member according to an example 4.
[Fig. 6]Fig. 6 illustrates a model for measuring a backscattering distribution to confirm a distribution of light that is backscattered and emitted.

### Description of Examples

A bioinformation acquisition apparatus will be described.

The bioinformation acquisition apparatus includes a light source for radiating a flux of light to a living body, i.e., a test object, a detector for detecting a signal output based on the radiation of the flux of light by the light source to the living body, and a reflective member for reflecting a flux of light emitted outside from the living body. A signal provided by the detector described above can be processed to measure a variety of biological information.

In doing so, the flux of light emitted outside from the living body when light is radiated to the living body can be reflected by the reflective member described above to be projected to the living body again, improving use efficiency of the radiated light. This will be described in detail later.

The detector used here may include photoacoustic measurement for detecting an acoustical wave signal using an ultrasonic detector, and ultrasonic optical modulation signal measurement in which a modulated optical signal provided by radiating an ultrasonic signal to a living body is detected using a light detector.

PAT measurement can be carried out when an acoustical wavedetector is provided to detect, as "a signal output based on radiation of a flux of light to a living body", an acoustical wave produced in a local area inside a test object due to irradiation of pulsed light. On the other hand, DOT measurement, AOT measurement and the like can be carried out when a light detector is provided to detect, as "a signal output based on radiation of a flux of light to a living body", weak light or light modulated by an ultrasonic wave which light has propagated and been diffused inside a living body.

DOT is the technology by which light is radiated from a light source to a living body, weak light which has propagated and been diffused inside the living body is sensed by a high-sensitive light detector, and an optical characteristic values distribution inside the living body is imaged from a sensed signal.

In Acousto-Optical Tomography (AOT), light is radiated into a living tissue, and at the same time a converged ultrasonic wave is radiated into a local area, and modulated light is detected by a light detector by using an effect in which light is modulated by an ultrasonic wave (acousto-optical effect). Also, AOT can detect X-ray phase variation in living tissue corresponding to absorption of optical energy.

Here, photoacoustic measurement using an acoustical wavedetector will be mainly described, but the present description can apply to various bioinformation acquisition apparatuses in which optical energy is required to be used efficiently.

A pulsed laser is mainly used as the light source for the photoacoustic measurement apparatus.

When a laser is used as the light source, Maximum permissible exposure (MPE) that is irradiation energy per unit area permitted to be applied onto the surface of a living body is provided by the International Code of laser usage. On the other hand, as a stronger optical energy is radiated to a living body, a stronger acoustical wave signal can be provided. Therefore, a wide area can be illuminated with permissible light exposure to radiate the large amount of optical energy.

A flux of light radiated into a living body is strongly diffused by cellular tissues and the like inside the living body and loses straightness, and a part thereof is backscattered and emitted outside from the living body.

When a flux of light is radiated to a uniform diffuse material having optical characteristics equivalent to those of a living body, most of the flux of light is backscattered due to diffusion effect in the living body and emitted out of the living body.

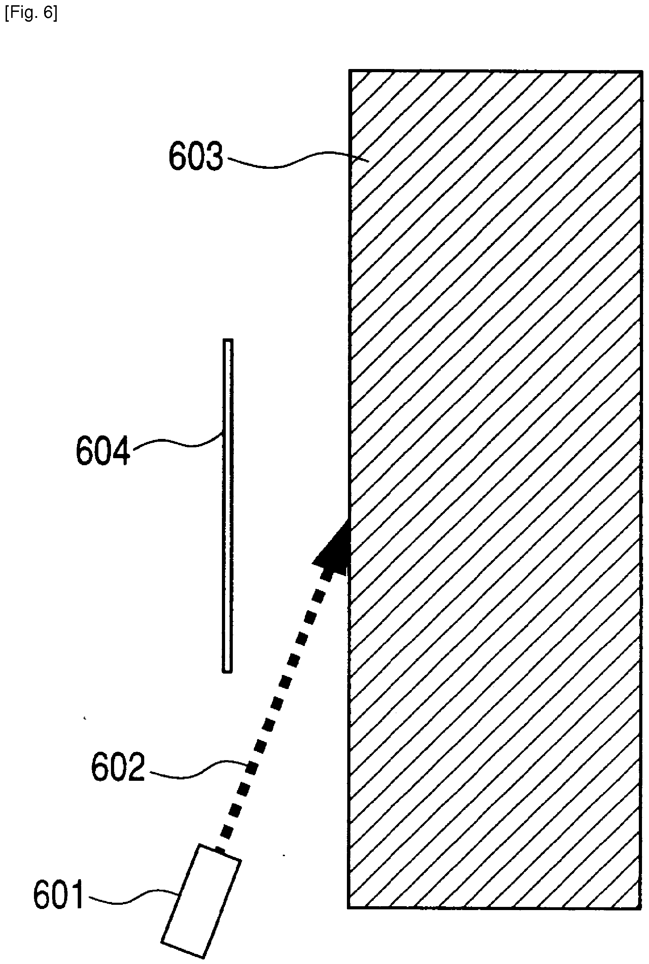

To confirm the above points, a distribution of backscattered and emitted light was checked by a ray tracing approach of Monte Carlo simulation using a model as shown in Fig. 6. Using a light source 601 with a diameter of 5 mm, a diffuse material 603 equivalent to a living body was irradiated obliquely with a flux of light 602 and a detection surface 604 was placed in front of an irradiation area. An emitted flux of light has an intense light quantity distribution directly above the irradiation area irrespective of an incident angle, and irradiates the detection surface at a certain spread angle depending on a diffuse factor. That is, the flux of light once entering a living body is diffused in the living body, and thus is emitted out of the living body in a range equal to or greater than the radiation area at an adequate angle.

The phrase "adequate angle" is used because light rays repeatedly scattered in a living body are spread to a certain extent and emitted out of the living body at a considerably wide angle by losing directivity although this depends on an optical constant such as a diffuse factor of the individual living body.

To re-radiate the emitted flux of light to the living body efficiently using a reflective member, the reflective member can be placed near the living body, above the irradiation area in the direction normal thereto. More desirably, the reflective member is placed over an area equal to or larger in size than the irradiation area to cover the entire irradiation area from above. When the reflective member is smaller in size than the irradiation area, although the emitted flux of light can be reflected and ejected at the living body again, only a reduced part of the flux of light can be reflected.

Also, to reflect the flux of light effectively, the reflective member needs to be placed very close to the irradiation area, imposing structural constraints.

The most suitable member with high reflectance can be used for the reflective member according to the wavelength of the light source used. In particular, Al and Au metals have high reflectance in the visible to near-infrared regions and thus are suitable for photoacoustic bioinstrumentation systems. Also, a dielectric single-layer or multilayer film may be used depending on the wavelength used.

Various shapes are available for a reflecting portion of the reflective member which reflects the flux of light emitted out of the living body.

Since the flux of light emitted out of the living body has a spread, to re-irradiate the initial irradiation area of the living body efficiently, desirably the reflecting portion has a concave shape. The use of the concave shape allows the spread flux of light to converge on a desired area. On the other hand, when the reflective member is placed close to the living body, the living body can be re-illuminated effectively even if the reflecting portion has a planar shape. Also, to illuminate a wider area of the living body including the area firstly illuminated, a planar reflecting portion can be used suitably.

The shape of the reflecting portion of the reflective member may be changed according to an emission angle of the flux of light emitted out of the living body. The emission angle depends on the diffuse factor which is based on living tissue. Thus, the emission angle also depends on such factors as measured region, age, and sex.

On the other hand, illumination distribution on the illuminated living body affects optical energy distribution of the flux of light propagating in the living body.

Photoacoustic signal strength is proportional to the intensity of optical energy at a location of an optical absorber. Therefore, to produce an accurate image of absorption coefficient intensity distribution of the optical absorber from a measured acoustical wave signal, it is desirable to clarify the optical energy distribution in the living body.

However, the optical constant of the living body is not uniform and it is difficult to determine the optical energy distribution. In particular, if there is a nonuniform illumination distribution on the surface of the living body, it is very difficult to clarify the accurate distribution of light in the living body.

If a laser beam ejected from the light source has a light flux distribution, the illumination distribution can be made uniform by designing the shape of the reflective member based on a known illumination distribution. Making the illumination light distribution on the surface of the living body uniform contributes to estimation of the optical energy distribution in the living body.

In a bioinformation measurement apparatus using photoacoustic effect, a measurement apparatus is proposed in which an illumination position from an ultrasonic detector differs relative to a living body which is a test object. That is, there may be cases where the illumination position is opposite to the ultrasonic detector relative to a living body, or the ultrasonic detector is disposed on the same side as an illumination area.

Particularly in the case where the ultrasonic detector is placed on the side of the illumination area, it is desirable from the viewpoint of illumination efficiency to radiate the flux of light to the surface of the living body facing the detection surface of the ultrasonic detector placed near the living body. In such an arrangement, the flux of light emitted out of the living body is emitted toward the front of the ultrasonic detector, and thus desirably the reflective member is placed between the ultrasonic detector and the irradiation surface. Also, in this case, a requirement for the reflective member is to be transparent to acoustical wave signals.

The reflective member transparent to acoustical waves is a member that transmits the acoustical waves with very low attenuation. Transparency to the acoustical waves can be ensured using a material with a low acoustic-wave attenuation factor for the member or, even when a material which tends to attenuate acoustical waves is used for the member, the member is sufficiently thin compared to the wavelength of the acoustical waves. The reflective member can be relatively increased in thickness when made of a dielectric film, but when a metal film which has versatility as a reflective film is used, it is necessary to control the thickness.

To detect an ultrasonic wave, an acoustically matching material is provided to ensure an acoustic match between the living tissue and the ultrasonic detector and the reflective member is placed in contact with the acoustically matching member. The living tissue has an acoustic impedance close to that of water, which is 1.5x10⁶ kg^{∗}m^{-2∗}s, and is about 1500 m^{∗}s⁻¹ at the velocity of sound.

The frequency of an ultrasonic detector used in the present invention is in the range of about 1-50 MHz, and the computed length of one wavelength is in the range of about 1.5 mm-30 µm.

When a material with high acoustic-wave attenuation is used for the reflective member, if the thickness of the material is set equal to or smaller than 1/30 the wavelength of the acoustical wave, transparency to the acoustical wave can be increased regardless of the material used. As a standard of transparency to an acoustical wave, the reflective member can have a thickness equal to or smaller than 1/30 the wavelength of the acoustical wave, but this is not a necessary condition. As described above, with a thickness equal to or larger than 1/30 the wavelength, the transmission factor for of the acoustical wave signal decreases gradually. However, if strength sufficient for a detection signal is available, the thickness may be equal to or larger than the one prescribed according to the present embodiment.

Thus, in living tissue, for a 10-MHz acoustical wave signal, whose one wavelength is about 150 um, desirably the thickness of the reflective member is 5 um or less.

### Examples

Examples will be described below.

### Example 1

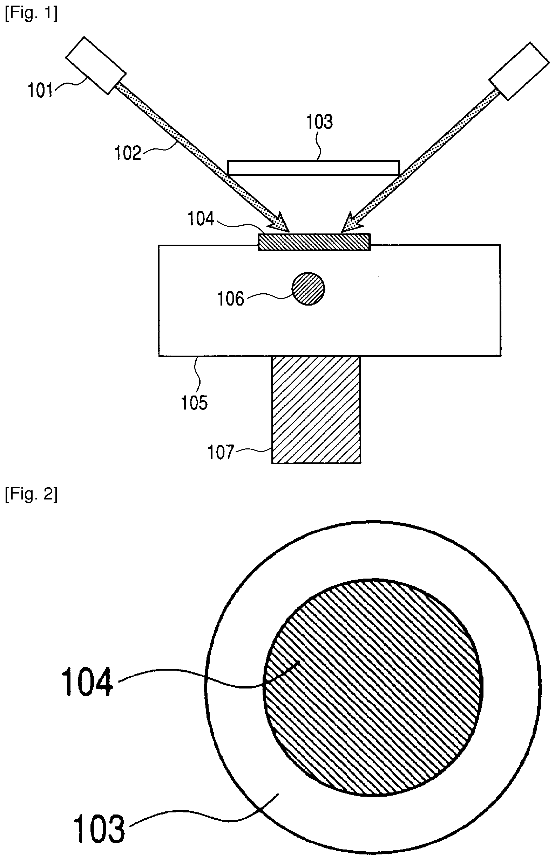

A configuration example of a bioinformation acquisition apparatus will be described with reference to Fig. 1, where the bioinformation acquisition apparatus is equipped with a reflective member. A flux of light 102 emitted from a light source 101 is radiated to a phantom 105 which simulates a living body. A reflective member 103 is placed close to the living body and just above an irradiation area 104 irradiated first.

Fig. 2 shows size relationship between an irradiation area and a reflective member. The reflective member 103 is larger than the irradiation area 104, and when viewed from above a surface of a test object, the reflective member 103 includes the irradiation area 201. The light source 101 is placed farther away from the irradiation area 104 than is the reflective member 103.

A 1064-nm Nd:YAG laser was used as the light source 101. The reflective member was a glass mirror prepared by coating a glass material with reflective films made of Au and Al as well as a dielectric multilayer film suitable for the wavelength of 1064 nm. A 10% aqueous solution of Intralipid was used as the phantom 105, having been prepared so as to have a uniform diffuse factor. A spherical optical absorber 106 was placed 2 cm away from the irradiation surface inside the Intralipid solution. An ultrasonic detector 107 was placed on the side opposite to the irradiation surface to measure a photo-acoustical wave signal.

Compared to when no reflective member was used, when the reflective member 103 was used, the photoacoustic signal strength was increased, and the photoacoustic signal was enhanced by increased quantity of radiated light.

### Example 2

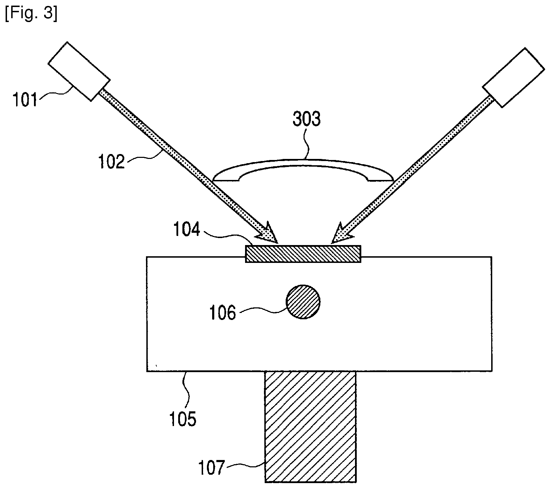

A configuration example of a bioinformation acquisition apparatus will be described with reference to Fig. 3, where the bioinformation acquisition apparatus is equipped with a reflective member. Except for a reflective member 303, the configuration of the present example is basically the same as the example 1.

In the present example, a concave mirror was used as a reflecting portion of the reflective member 303. It was observed that the photo-acoustical wave signal was intensified remarkably compared to when no reflective member was used. This is believed to be because the reflecting portion of the reflective member, which is concave in shape, allows the light scattered from the living body with an extent to be re-radiated efficiently toward the vicinity of the irradiation area.

In particular, when the reflective member 303 was placed away from the irradiation area 104, it was observed that the photoacoustic signal was enhanced more remarkably than with the reflective member equipped with the planar reflecting portion according to the example 1.

### Example 3

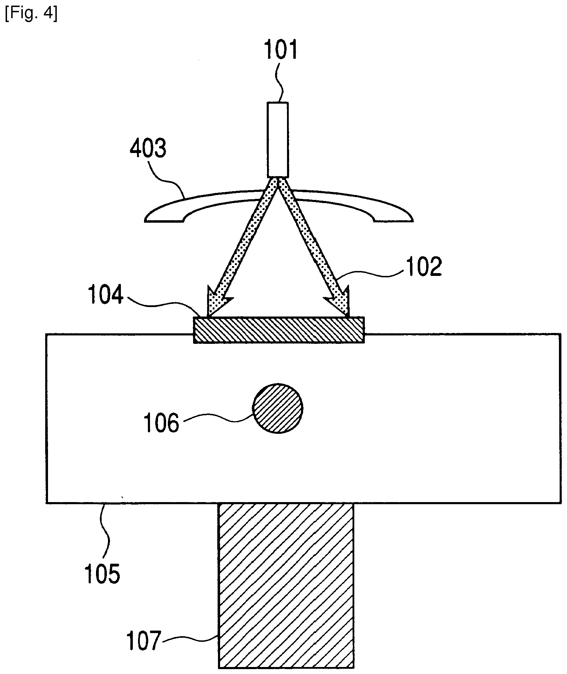

A configuration example of a bioinformation acquisition apparatus will be described with reference to Fig. 4, where the bioinformation acquisition apparatus is equipped with a reflective member. In the present example, a hole is provided in part of a reflecting portion of a reflective member 403 and the flux of light 102 is radiated to a test object 105 through the hole. Otherwise the configuration of the present example is basically the same as the example 2.

Since a top surface of the irradiation area 104 is not covered entirely by the reflective member 403, enhancement effect on the photo-acoustical wave signal was reduced, but a marked effect was observed compared to when no reflective member was used.

The use of such a reflective member allows incident light to illuminate the living body at an angle close to vertical, enabling the use of various types of photoacoustic measurement apparatus.

Although in the exemplary embodiment 3, a single hole is provided in the reflective member 403, a number of holes may be provided through which a number of branched beams produced by optical fiber or the like is radiated. Besides, needless to say, it is desirable that the hole has a small size. Thus, the flux of light may be focused through the hole in the reflective member 403 once before illuminating the test object 105.

### Example 4

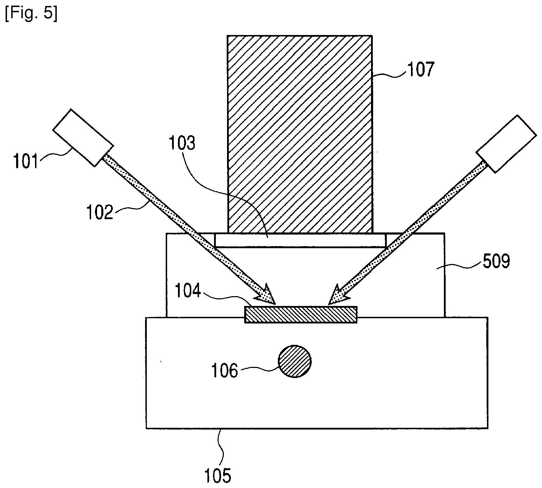

A configuration example of a bioinformation acquisition apparatus will be described with reference to Fig. 5, where the bioinformation acquisition apparatus is equipped with a reflective member. In the present unclaimed embodiment, the ultrasonic detector 107 and the light source 101 were placed on the same side of the test object 105. The reflective member 103 was placed between the ultrasonic detector 107 and the irradiation area 104. In such an arrangement, the reflective member needs to have the property of reflecting the flux of light, but transmitting acoustical waves therethrough. Therefore, an Al film of 5 microns in film thickness was deposited on a resin film with low acoustic-wave attenuation to produce the reflective member 103, which was then mounted on the front of the ultrasonic detector 107.

To radiate the flux of light 102 to the test object 105 located below the ultrasonic detector 107, a standoff made of an acoustically matching material 509 was provided.

Compared to when no reflective member was used, the photo-acoustical wave signal was enhanced and it was observed that the placement of the reflective member had the effect of increasing the quantity of radiated light.

It is to be understood that the invention is not limited by the disclosed examples. The invention is defined by the appended claims.

## Claims

1. A bioinformation acquisition apparatus configured for photoacoustic measurement, comprising:
a light source (101) configured for radiating a flux of light (102) to form an irradiation area (104) on a test object (105);
a detector (107) configured for detecting an acoustical wave signal generated in the test object (105) based on radiation of the flux of light (102) to the test object (105); and
a reflective member (103; 303; 403), having a concave shape, adapted to reflect a flux of light (102) emitted out of the test object (105) when the flux of light (102) is radiated to the test object (105),
wherein the light source and the detector are located at the same side with respect to the irradiation area (104) on the test object (105), and
wherein the reflective member (103; 303; 403) has a thickness equal to or smaller than 5 µm and is located between the detector (107) and the irradiation area (104) on the test object (105) so as to transmit the acoustic wave, and is configured to be equal to or larger in size than the irradiation area (104).

2. The bioinformation acquisition apparatus according to claim 1, wherein:
the radiation of the flux of light (102) to the test object (105) is oblique irradiation of the surface of the test object (105); and
the reflective member (103; 303; 403) is placed so as to cover an entire top surface of the irradiation area (104; 201) without blocking the flux of light (102) produced by the oblique irradiation.

3. The bioinformation acquisition apparatus according to claim 1 or 2, wherein the reflective member (103; 303; 403) is made of a metal or a dielectric.

## Patentansprüche

1. Vorrichtung zur Erfassung von Bioinformationen, gestaltet für eine photoakustische Messung, mit:
einer Lichtquelle (101), die gestaltet ist, um einen Lichtstrom (102) auszustrahlen, um einen Bestrahlungsbereich (104) auf einem Testobjekt (105) zu bilden;
einem Detektor (107), der gestaltet ist, um ein akustisches Wellensignal zu erkennen, das in dem Testobjekt (105) erzeugt wird, basierend auf einer Ausstrahlung des Lichtstromes zu dem Testobjekt (105); und
einem reflektierenden Bauteil (103; 303; 403), das eine konkave Form hat, die so angepasst ist, dass sie einen Lichtstrom (102) reflektiert, der von dem Testobjekt (105) her emittiert wird, wenn der Lichtstrom (102) zu dem Testobjekt hin ausgestrahlt wird,
wobei sich die Lichtquelle und der Detektor auf derselben Seite befinden, mit Bezug auf den Bestrahlungsbereich (104) auf dem Testobjekt (105), und
wobei der reflektierende Bereich (103; 303; 403) eine Dicke gleich oder kleiner als 5 µm hat und sich zwischen dem Detektor (107) und dem Bestrahlungsbereich (104) auf dem Testobjekt (105) befindet, um die akustische Welle zu übermitteln, und so gestaltet ist, dass er gleich groß oder größer als der Bestrahlungsbereich (104) ist.

2. Vorrichtung zur Erfassung von Bioinformationen nach Anspruch 1, wobei:
die Ausstrahlung des Lichtstromes (102) zu dem Testobjekt (105) eine indirekte Einstrahlung auf die Fläche des Testobjektes (105) ist; und
das reflektierende Bauteil (103; 303; 403) so angeordnet ist, dass es die gesamte obere Fläche des Bestrahlungsbereiches (104; 201) abdeckt, ohne den Lichtstrom (102) zu blockieren, der von der indirekten Einstrahlung erzeugt wird.

3. Vorrichtung zur Erfassung von Bioinformationen nach Anspruch 1 oder 2, wobei das reflektierende Bauteil (103; 303; 403) aus einem Metall oder Dielektrikum hergestellt ist.

## Revendications

1. Appareil d'acquisition d'informations biologiques configuré pour une mesure photo-acoustique, comprenant :
une source de lumière (101) configurée pour projeter un rayonnement d'un flux de lumière (102) pour former une zone d'exposition à un rayonnement (104) sur un objet test (105) ;
un détecteur (107) configuré pour détecter un signal d'onde acoustique généré dans l'objet test (105) sur la base d'un rayonnement du flux de lumière (102) vers l'objet test (105) ; et
un élément réfléchissant (103 ; 303 ; 403), ayant une forme concave, conçu pour réfléchir un flux de lumière (102) émis en sortie de l'objet test (105) lors de la projection d'un rayonnement du flux de lumière (102) vers l'objet test (105),
dans lequel la source de lumière et le détecteur sont situés du même côté par rapport à la zone d'exposition à un rayonnement (104) sur l'objet test (105), et
dans lequel l'élément réfléchissant (103 ; 303 ; 403) a une épaisseur égale ou inférieure à 5 µm et est situé entre le détecteur (107) et la zone d'exposition à un rayonnement (104) sur l'objet test (105) de façon à transmettre l'onde acoustique, et est configuré pour avoir une taille égale ou supérieure à celle de la zone d'exposition à un rayonnement (104).

2. Appareil d'acquisition d'informations biologiques selon la revendication 1, dans lequel :
la projection de rayonnement du flux de lumière (102) vers l'objet test (105) correspond à une exposition à un rayonnement oblique de la surface de l'objet test (105) ; et
l'élément réfléchissant (103 ; 303 ; 403) est placé de façon à couvrir toute une surface supérieure de la zone d'exposition à un rayonnement (104 ; 201) sans occulter le flux de lumière (102) produit par l'exposition à un rayonnement oblique.

3. Appareil d'acquisition d'informations biologiques selon la revendication 1 ou 2, dans lequel l'élément réfléchissant (103 ; 303 ; 403) est constitué d'un métal ou d'un diélectrique.
